## Europäisches Patentamt

## European Patent Office

## Office européen des brevets

(19)

(11) Publication number: **0 048 521**
A2

(12) **EUROPEAN PATENT APPLICATION**

(21) Application number: 81201042.9

(22) Date of filing: 17.09.81

(51) Int. Cl.³: **C 12 N 9/58**
C 12 N 1/14, A 23 C 19/032
//C12R1/785

(30) Priority: 22.09.80 US 189166

(43) Date of publication of application:
31.03.82 Bulletin 82/13

(84) Designated Contracting States:
AT BE CH DE FR GB IT LI LU NL SE

(71) Applicant: Gist - Brocades N.V.
Wateringseweg 1 P.O. Box 1
NL-2600 MA Delft(NL)

(72) Inventor: Joyeaux, André
rue Eugene Jacquet 36
Marcq en Baroeul(FR)

(74) Representative: Huygens, Arthur Victor, Dr.
c/o Gist-Brocades N.V. Patent & Trademarks
Department Wateringseweg 1 PO-box 1
NL-2600 MA Delft(NL)

(54) Novel fungus of Mucor miehei and its use in obtaining a new milk-clotting enzyme.

(57) A novel fungus of the species Mucor miehei designated as NRRL 12268 and its variants and mutants capable of producing high yields of enzyme product with a high milk coagulating activity and low proteolytic activity and the enzyme product produced by growing under aerobic conditions of a culture of Mucor meihei NRRL 12268 in a medium containing appropriate nutrients and recovering the resulting enzyme, preferably free of lipase as well as a process for producing cheese by coagulating milk with the said enzyme.

EP 0 048 521 A2

Croydon Printing Company Ltd.

## Novel fungus of Mucor miehei and its use in obtaining a new milk-clotting enzyme.

The invention relates to a novel fungus, Mucor miehei NRRL 12268, and to a novel method of obtaining a milk-clotting enzyme by cultivation of the said fungus or its variants or mutants and the enzyme product produced thereby.

The invention relates further to an improved process of producing cheese by coagulating milk with the novel enzyme.

The conventional method of making cheese involves the use of rennet for coagulating milk. Rennet is an enzyme-containing composition prepared from the fourth stomach of milk-fed calves. In the process of making cheese, the rennet is added to milk and the enzyme, rennin, exerts a mildly proteolytic action on the casein and other proteins present in the milk. This breakdown of the proteins causes the milk to coagulate or to form solid curds. These curds are separated from the whey, which is predominantly an aqueous suspension of low solids content. The curds are then mixed with salt, etc., and formed into blocks or rounds and cured to form cheese.

Because of the particular animal origin of rennet, the supply and quality of rennet are subject to wide fluctuations. In view of these variable factors, investigators in the field have attempted to find cheese making substitutes for rennet. Numerous vegetable and microbial enzyme preparations have been investigated in the search for suitable substitute materials having the milk coagulating properties of rennet. Among the microorganisms which have been disclosed as useful for this purpose are, for example, the various microorganisms disclosed in U.S. Pat. No. 1,391,219, the Endothia parasitica disclosed in U.S. 3,275,453 and the Mucor pusillus disclosed in U.S. Pat. Nos. 3,151,039 and 3,212,905.

U.S. Patents Nos. 4,136,201 (reissued as Re 30,669) and No. 3,988,207 describe Mucor miehei which will produce an enzyme useful for the coagulation of milk in cheese making processes and U.S. Patent No. 4,136,201 describes a lipase-free enzyme derived from Mucor miehei capable of producing a wide variety of cheeses by coagulation of milk.

As result of extensive research and experimentation an improved process for the production of a milk-clotting enzyme with low proteolytic activity in a significantly enhanced yield was surprisingly found. The present invention accordingly provides a process for the preparation of a microbial rennin having high milk-coagulating activity and low proteolytic activity which comprises cultivating under aerobic conditions, Mucor miehei NRRL 12268 or a productive variant or mutant thereof in a culture medium containing assimilable carbon, nitrogen and trace nutrients and recovering the microbial rennin from the culture medium.

A description of the general characteristics of the fungus of Mucor miehei is provided by Cooney and Emerson in their book Thermophilic Fungi, p. 17-27 (1964) published by W.H. Freeman and Co, San Francisco and London. The specific Mucor miehei of the present invention has been deposited with the Northern Regional Research Laboratories, Peoria, Ill. and has been given the identification No. NRRL 12268. The said deposit has been made in accordance with the provisions of Section 608.01 (p) of the Manual of Patent Examining Procedure and with the provisions of Rule 28 of the European Patent Convention.

The Mucor miehei NRRL 12268 was prepared by subjecting Mucor miehei strain NRRL A-13042 to alkylating agents such as ethyl methylsulphonate (EMS) and selection by plating on an agar casein medium and looking for the clones giving the largest clotting zones and further selecting the micro-organisms by cultivation in a liquid medium, such as those described in US Patent No. 4,136,201.

The Mucor miehei strain NRRL 12268 shows morphological and taxonomical characteristics, which are rather similar to those of the prior art Mucor miehei strains, e.g. NRRL A-13042, NRRL A-13131, NRRL A-7772 and NRRL no. 3169.

The microbial rennin of the present invention preferably is prepared by growing a culture of the selected NRRL 12268 Mucor miehei strain or a variant or mutant thereof in a suitable medium in the presence of air at temperatures of from about 30°C to about 55°C for periods of time of from about 2 to about 14 days. The fermentation medium generally has a pH of from about 3 to about 8 and preferably a pH of from about 4 to about 7. Submerged aerobic fermentation methods, for example, deep fermentation in commercial fermentation tanks or fermentation in flasks on a rotary shaker, as well as various methods of surface aerobic fermentation can be used to prepare the microbial rennin of the present invention. Submerged aerobic fermentation is preferred.

According to a further aspect of the invention the thermal sensitivity of the microbial rennin may be improved by treating the rennin with an oxidizing agent in a manner known per se. The process may be applied to as well a filtrate derived by recovery of the fermentation broth, or a more or less concentrated filtrate, obtained by evaporation under vacuo or ultrafiltration. Suitable oxidizing agents are, for example, salts of trichlorocyanuric acid, periodic acid, hypochlorous acid, chloric acid and iodic acid and preferably the sodium, potassium or calcium salts. A preferred oxidizing agent is sodium hypochlorite.

It was surprisingly found that when using the novel strain, a lower viscosity of the fermentation medium is obtained. According to a further feature of the invention the fermentation may be carried out in the presence of ammonium sulphate in order to lower the viscosity of the fermentation medium. It will be appreciated that such lower viscosities represent attractive advantages, such as less power and energy for mixing, better transfer and easier recovery.

Recovery can be carried out by simple solid-liquid separation such as filtration or centrifugation, whereby most of the desired enzyme is found in the liquid phase or by simple concentration of the liquid phase by evaporation, ultrafiltration or by other methods conventionally used in the separation of proteins from mixtures, for example, solvent precipitation, salt precipitation, chromatography and other such methods of protein recovery. Examples of solvents that can be used for the precipitation of the microbial rennin are ketones such as acetone and methyl ethyl ketone, alcohols such as methanol, ethanol and isopropanol, and other such organic solvents. Illustrative of the salts which can be employed are ammonium sulphate, sodium sulphate and the like mineral salts. Still other methods of recovery of the microbial rennin can be used, for example, dialysis, electrophoresis and freeze-drying.

Suitable fermentation media can be prepared from carbohydrate materials such as whey, degraded corn starch, Cerelose, wheat bran and other organic sources of available carbon, and from nitrogenous materials such as brewers' yeast, soya protein, casein, urea, ammonium salts, nitrates and other such organic or inorganic sources of available nitrogen. The sources of these fermentation media ingredients may be crude natural materials or more highly purified substances. Trace nutrients that may be required by the organism are usually present with the major fermentation media ingredients, such that the separate addition of trace nutrients is generally not required. For example, trace amounts of inorganic salts such as metal chlorides, sulphates, phosphates and nitrates are generally present in the fermentation media in association with the carbonaceous and nitrogenous ingredients.

Proteases, in general, coagulate milk. However, most proteases cause considerable protein digestion. When these proteases, of animal, plant and microbial origin, are employed for the coagulation of milk, the resulting curds develop undesirable off-flavors due to proteolysis and thus are not well suited for cheese making. A principal advantage of the present invention is that the rennin prepared from the Mucor miehei as herein described unexpectedly has a very low proteolytic activity associated with high milk coagulating activity. The milk proteolysis curves obtained with the Mucor miehei of the present invention are identical to those obtained with rennet, which indicates a very low proteolytic activity.

In the production of certain cheese it is desirable to use a microbial rennin obtained from Mucor miehei in which the rennin is essentially free from any lipase and/or esterase whereas in the production of certain other cheese it may be desirable to use a microbial rennin obtained from Mucor miehei in which the rennin contains some lipase and/or esterase. It will be understood that both of the aforesaid microbial rennins are included within the scope of the present invention although a lipase-free product is preferred. A microbial rennin containing lipase obtained by the process of the invention can be treated in known manner, for example by heating at 60°C, to obtain a lipase-free product.

The activity of the microbial rennin of the invention may be determined by the SRA method carried out as follows:

Substrate: 10 % non-fat dried milk

90 % 0.01 M $CaCl_2$      5.0 ml

Enzyme:      0.5 ml

Temperature: 25° C

Observe time required for appearance of first floc.

Range: 100 - 600 second floc time.

DERIVATION OF EQUATION Definition: SRA = units of rennin act/g.

A unit of rennin activity is defined as 1/100 the activity of 1 g Rennet extract - Standard Single Strength (Hansen).

SRA of standard rennet extract = 100 or 1 SRA unit is the activity of 10 mg of standard rennet extract.

$$SRA = K \frac{1}{(sec. floc. time)}/g$$

Let X = seconds foc. time

Plot of $\frac{1}{X} \cdot 10^5$ vs. mg Standard rennet extract showed:

a) line intersects the Y axis at 50 $\frac{1}{sec.} \cdot 10^5$ above the origin

a correction (C) is required.

$$SRA = (K) (\frac{1}{X} \cdot 10^5) - C/g$$

b) Slope of line = 153 $\frac{1}{sec.} \cdot 10^5/mg$

$$mg = \frac{\frac{1}{X} \cdot 10^5 - 50}{153}$$

$$mg = 654 (\frac{1}{X}) - 0.33$$

$$SRA = 65,400 (\frac{1}{X}) - 33/mg$$

Working equation

SRA = 65,400/sec. floc. time - 33/mg .

In the following examples there are described several preferred embodiments to illustrate the invention. However it should be understood that the invention is not intended to be limited to the specific embodiments and that various modifications of the compositions and processes may be made without departing from the spirit or scope of the invention.

## EXAMPLE 1

A mash composition consisting of 90.8 g of glucose, 4.5 kg of Casein hydrolysate, 0.45 kg of yeast extract and sufficient water for a total weight of 454 kg was added to an inoculum tank and the pH was adjusted to 5.3 to 5.7. The liquid was then sterilized by heating at 130-131°C for 45 to 50 minutes and then the mixture was cooled to 35.6 to 37.2°C. The mixture was then inoculated with 0.1% (Vol./Vol.) of a preculture of Mucor miehei NRRL 12268 containing the same main ingredients and the inoculum was allowed to grow at the said temperature with stirring while passing air therethrough for 20 to 50 hours.

A fermentor tank was charged with a mash comprising 74 kg of pearl starch, 18.6 kg of soy flour, 9.5 kg of whey solids, 0.06 kg of soy bean oil, sufficient spray dried bacterial alpha-amylase to provide liquefaction of starch and sufficient water for a total weight of 454 kg. After starch degradation the mash was sterilized by heating at 130 to 133°C for 45 to 50 minutes and the pH was adjusted to 5.6 to 5.8. The mash was then inoculated with 1 to 5% by volume of the inoculum produced above and fermentation was conducted at 36.7 to 38.9°C with stirring and air flow therethrough for 100 to 140 hours. During the fermentation an anti-foaming agent was added to reduce foaming in the fermentors and 1.3% by weight of ammonium sulphate as a 25% aqueous solution was added. More preferably such an ammonium sulphate solution is added depending on the adjustment of an appropriate viscosity level.

The mash was removed from the fermentor and was vacuum filtered to remove the cell debris from the liquid and the filtrate was then subjected to ultra filtration through a semi-permeable membrane to obtain a concentrated extract containing the enzyme. The extract was concentrated to obtain the desired concentration of enzyme in the liquid and the pH was adjusted to about 4. The mixture was then heated to about 60°C for a time sufficient to inactivate lipase and esterase without adversely effecting the enzyme.

A cheese was prepared by substituting for the normal rennet preparation used in the setting step, an equivalent amount of microbial rennin product produced above. In this procedure, pasteurized milk is adjusted to 30 to 31.1°C and one percent by weight of a commercial lactic acid starter solution was added. The microbial rennin was then added to the milk at a rate of 0.085 kg adjusted to a potency equivalent of single strength extract per 454 kg of milk. The mixture was agitated until a curd of satisfactory firmness was obtained. The curd was cut into cubes and then cooked at 37.7°C for several hours. The curd was separated from the whey and layered into slabs. The milled curd was then salted with 3% by weight of cheese salt. The salted curd was transferred to hoops, pressed, and then placed in a curing room. The cheese made from the microbial rennin of this example was sampled periodically after several days of curing and was found to possess excellent qualities and to be essentially free from off-flavors.

Other conventional methods of making Cheddar cheese, for example those generally described by Prescott and Dunn, "Industrial Microbiology", Chapter 21 (3rd ed. 1959), McGraw-Hill Book Co., Inc. New York, and references cited therein, can be used in the practice of the present invention by substitution for the rennet preparations ordinarily employed in said cheese making, an equivalent amount of the microbial rennin from Mucor miehei of Example 1 herein to produce good quality cheese.

EXAMPLE 2

Using the procedure of Example 1, a comparison was conducted using a prior art Mucor miehei designated as M-67 and Mucor miehei NRRL 12268 and the results are reported in

Table I.

TABLE I

| Strain | Fermentation time | % of $(NH_4)_2SO_4$ | Yield in SRA/g |
|---|---|---|---|
| M-67 | 68 h | 1.0 | 21.7 |
| NRRL 12268 | 52 h | 1.0 | 33.4 |
| M-67 | 50-54 h | 1.3 | 26.0 |
| NRRL 12268 | 41-46 h | 1.3 | 42.0 |
| | 38-45 h | 1.3 | 54.2 |
| | 38-42 h | 1.3 | 54.5 |
| | 33-39 h | 1.3 | 69.7 |

The results of Table I show the clear superiority of the novel Mucor miehei NRRL 12268 for the production of microbial rennin as compared to the prior art fungus.

The strain M-67 is a descendant of the parent strain NRRL A-13042 obtained by normal cultivation and selection procedures.

EXAMPLE 3

Using the procedure of Example 1, a comparison was conducted using a prior art Mucor miehei designated as M-67 and Mucor miehei NRRL 12268 and the results are reported in Table II.

The viscosity of fermented mash is given in Pascal seconds (Pa.s.).

TABLE II

| Fermentation time | Strain M-67 | NRRL 12268 |
|---|---|---|
| 40 h | 0.300 Pa.s | 0.025 Pa.s |
| 70 h | 0.800 Pa.s | 0.180 Pa.s |

- 9 -

It is obvious that the novel strain gives a fermented mash with lower viscosity and thus with the consequences well known from the man of the art: less power for mixing, better transfer, easier recovery.

## EXAMPLE 4

Using the procedure of Example 2, a comparison was conducted using the new strain NRRL 12268 to demonstrate the influence of the addition of ammonium sulphate solution on mash viscosity in comparison with the same amount of water.

TABLE III

| Fermentation time | Add. of water at 66 hours | Add. of $NH_4(SO_4)_2$ 1.3% at 66 hours |
|---|---|---|
| 42 hours | 0.012 Pa.s | 0.021 Pa.s |
| 49 hours | 0.026 Pa.s | 0.034 Pa.s |
| 66 hours | 0.215 Pa.s | 0.092 Pa.s |
| 66 hours, after add. | 0.182 Pa.s | 0.074 Pa.s |
| 94 hours | 0.400 Pa.s | 0.084 Pa.s |
| 114 hours | 0.504 Pa.s | 0.126 Pa.s |

## Claims

1. A process for the preparation of a microbial rennin having high milk-coagulating activity and low proteolytic activity which comprises cultivating under aerobic conditions Mucor miehei NRRL 12268 or a productive variant or mutant thereof in a culture medium containing assimilable carbon, nitrogen and trace nutrients and recovering the microbial rennin from the culture medium.

2. A process according to claim 1 in which the culture is carried out at 30°C to 55°C for from 2 to 14 days.

3. A process according to claim 1 or 2 in which the pH of the culture medium is from 4 to 7.

4. A process according to claim 1 in which the culture is carried out by submerged aerobic fermentation.

5. A process according to any one of the preceding claims in which a mixture of enzymes obtained is treated in known manner to produce a lipase-free microbial rennin.

6. A process according to any one of the preceding claims which comprises adding to the fermentation medium ammonium sulphate to reduce the viscosity of the medium.

7. A process according to any one of the preceding claims which comprises treating an aqueous microbial rennin containing liquid with an oxidizing agent to reduce the thermal sensitivity of the rennin.

8. A microbial rennin having high milk-coagulating activity and low proteolytic activity obtained by a process according to any one of claims 1 to 7.

9. A microbial rennin according to claim 8 which is substantially free from lipase.

10. A microbial rennin according to claim 8 of reduced thermal sensitivity obtained by a process according to claim 7.

11. A culture of Mucor miehei NRRL 12268 or a variant or mutant thereof capable of producing high yields of an enzyme product with a high milk-coagulating activity and low proteolytic activity, in a synthetic culture medium containing sources of assimilable carbon, nitrogen and trace nutrients, and substantially free from other micro-organisms.

12. Mucor miehei NRRL 12268 or a variant or mutant thereof capable or producing high yields of an enzyme product with a high milk-coagulating activity and low proteolytic activity.

13. A process for making cheese which comprises the preparation of curds from milk, in which at least part of the rennet normally used to prepare the curds is replaced by a microbial rennin as claims in claimed in any one of claims 8 to 10.